(19) Europäisches Patentamt
European Patent Office
Office européen des brevets

(11) Numéro de publication : **0 112 770 B1**

(12) # FASCICULE DE BREVET EUROPÉEN

(45) Date de publication du fascicule du brevet : **01.10.86**

(51) Int. Cl.⁴ : **A 61 K 35/78**, C 07 D313/08

(21) Numéro de dépôt : **83402463.0**

(22) Date de dépôt : **19.12.83**

(54) **Nouvel extrait de ruscus, son procédé de préparation et son application en tant que principe actif veinotonique.**

(30) Priorité : **28.12.82 FR 8221899**

(43) Date de publication de la demande : **04.07.84 Bulletin 84/27**

(45) Mention de la délivrance du brevet : **01.10.86 Bulletin 86/40**

(84) Etats contractants désignés : **AT BE CH DE FR GB IT LI LU NL SE**

(56) Documents cités : **EP-A- 0 067 086**

(73) Titulaire : **PIERRE FABRE S.A.**
**125, rue de la Faisanderie**
**F-75116 Paris (FR)**

(72) Inventeur : **Hatinguais, Philippe**
**Le Landou**
**F-81100 Castres (FR)**
Inventeur : **Patoiseau, Jean-FranCois**
**7 rue Jules Ferry**
**F-81100 Castres (FR)**
Inventeur : **Marcelon, Gilbert**
**Route de Castres Sémalens**
**F-81700 Castres (FR)**
Inventeur : **Negol, Paul**
**Route de Laprade**
**F-81290 Labruguiere (FR)**

(74) Mandataire : **Schrimpf, Robert et al**
**Cabinet Regimbeau 26, Avenue Kléber**
**F-75116 Paris (FR)**

EP 0 112 770 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention, réalisée au Centre de Recherche Pierre Fabre, concerne un procédé de préparation de nouveaux extraits de Ruscus (Liliacées), et en particulier de Ruscus aculeatus, utiles en thérapeutique dans le domaine cardiovasculaire.

Il est bien connu que les substances stéroliques du Ruscus aculeatus, c'est-à-dire les Ruscogénines et les saponines correspondantes, sont douées de propriétés veinotoniques, et constituent d'ailleurs les principes actifs de diverses spécialités pharmaceutiques.

Dans la technique antérieure, aucun autre principe actif n'a pu être localisé dans un extrait de Ruscus, et en particulier de Ruscus aculeatus. On a toujours considéré jusqu'à ce jour que les propriétés thérapeutiques de ces extraits connus étaient directement liées à la présence des hétérosides stéroliques, ou des produits issus de leur hydrolyse.

L'état de la technique peut en outre être complété par EP-A-0 067 086 qui décrit de nouveaux composés de la série des benzoxépines ainsi que leurs analogues soufrés et azotés. En ce qui concerne la préparation de ces composés, seule une synthèse organique est préconisée par ce document.

De façon tout à fait surprenante, la demanderesse a découvert à présent que certaines fractions extraites de Ruscus, bien qu'étant totalement exemptes de saponines et de génines, possédaient néanmoins des propriétés veinotoniques remarquables qui seront évoquées plus en détail ci-après. La demanderesse a ainsi découvert que la technique antérieure d'extraction du Ruscus qui conduisait à l'obtention de composés stéroliques à base de ruscogénine, néoruscogénine et composés apparentés, n'était donc pas la mieux appropriée à l'obtention de principes actifs dans le système cardiovasculaire.

Conformément à la technique antérieure, les extraits de Ruscus étaient obtenus par épuisement des rhizomes par l'eau ou par des solutions hydroalcooliques, et la purification éventuelle avait pour seul but un enrichissement en composés stéroliques par exemple par extraction liquide-liquide en milieu alcalin ou relargage par le sulfate d'ammonium.

La demanderesse a découvert à présent qu'en mettant en œuvre un nouveau procédé d'extraction, il était possible d'obtenir un extrait de Ruscus, et en particulier de Ruscus aculeatus, fondamentalement différent des extraits de la technique antérieure. En effet, conformément à la présente invention, l'extrait de Ruscus est exempt de saponines stéroliques et contient d'environ 0,5 % à environ 5 % en poids de ruscobenzoxépine de formule

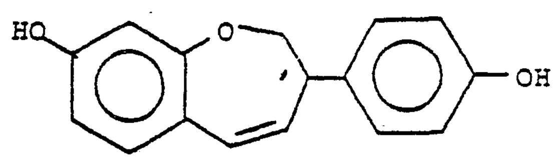

Conformément à la présente invention, le procédé de préparation d'un extrait de Ruscus, et en particulier de Ruscus aculeatus, se trouve caractérisé par le fait qu'il comporte au moins une première étape d'extraction par un alcool ou un mélange hydroalcoolique, suivie d'une évaporation et d'une seconde étape d'extraction par un ester ou une cétone, en vue d'éliminer les saponines stéroliques présentes dans l'extrait résultant de la première étape.

Selon une caractéristique particulière de la présente invention, la première étape d'extraction peut avantageusement être mise en oeuvre par épuisement des rhizomes de Ruscus aculeatus avec de l'éthanol à 95-99 %. Il est cependant parfaitement possible d'effectuer cette première étape d'extraction à l'aide d'alcool constitué par des alcanols en $C_1$ à $C_7$ ou par des mélanges hydroalcooliques en proportions diverses, mais contenant cependant de préférence une fraction prépondérante d'un alcanol en $C_1$ à $C_7$. L'opération d'évaporation du solvant alcoolique ou hydroalcoolique contenant le principe actif peut être réalisée de façon classique par exemple par évaporation sur une matière inerte et/ou absorbante telle que la silice, l'alumine, un carbonate alcalin ou alcalino-terreux, des sucres, du kaolin, de la cellulose, ou de la cyclodextrine.

La seconde étape d'extraction, après évaporation du solvant alcoolique ou hydroalcoolique, est réalisée par reprise de l'extrait sec ainsi obtenu par exemple dans de l'acétate d'éthyle. Dans pareil cas, l'extractible à l'acétate d'éthyle ne représente que 10 à 20 % de l'extrait alcoolique résultant de la première étape, soit un maximum de 3 à 4 % environ des rhizomes de départ.

Il est clair que des résultats parfaitement satisfaisants peuvent également être obtenus en mettant en œuvre, pour la première étape d'extraction, des solvants de polarité voisine de l'éthanol, tels que le méthanol, le propanol ou le butanol.

Par ailleurs, dans la pratique l'acétate d'éthyle a également pu être remplacé sans aucun inconvénient majeur par d'autres esters tels que l'acétate d'isopropyle et l'éther isopropylique ou encore par des cétones telles que l'acétone, la diméthylcétone, la méthyléthylcétone ou la méthylisobutylcétone.

L'exemple rapporté ci-après concerne un procédé mis en œuvre avec l'éthanol et l'acétate d'éthyle respectivement pour la première et la seconde étapes d'extraction, car ce sont des solvants usuels non toxiques.

Les extraits ainsi obtenus, à la suite de la seconde étape d'extraction par un ester ou une cétone, peuvent encore être purifiés par traitement à la silice ou à l'alumine, soit en cuve, soit par chromatographie sur colonne. Un tel traitement permet d'isoler les fractions les moins polaires de l'extrait.

A titre d'exemple non limitatif, 10 g de l'extrait obtenu à la suite de la seconde étape d'extraction, par exemple à l'acétate d'éthyle, sont chargés sur 250 g de silice et l'élution est réalisée par un mélange ternaire chloroforme-méthanol-eau. On remarquera à ce propos que les principes actifs recherchés dans le nouvel extrait de Ruscus conformément à la présente invention, ont des propriétés phénoliques, par conséquent, toutes les opérations mettant en œuvre les extractions liquide-liquide en milieu alcalin, puis acide, les résines échangeuses d'ions ou la poudre de polyamide ou polyvinylpyrrolidone contribueront à la purification de l'extrait.

## Exemple

100 kg de parties souterraines de Ruscus aculeatus sont épuisés par 500 litres d'éthanol à 95° G.L à ébullition pendant 2 heures, et après filtration du solvant, le végétal est soumis à une deuxième extraction dans les mêmes conditions. Les solutions alcooliques sont concentrées à sec, poids : 15 kg environ (produit A).

12 kg de produit A sont mis en suspension dans 120 litres d'acétate d'éthyle et portés à ébullition pendant 6 heures. L'insoluble est filtré et repris dans 60 litres d'acétate d'éthyle dans les mêmes conditions. Les solutions organiques réunies sont évaporées sous vide. On obtient ainsi un résidu à consistance de miel, poids : 2,5 kg environ (produit B).

Une purification complémentaire est effectuée par chromatographie de 250 g du produit B sur une colonne de silice éluée par le mélange chloroforme-méthanol-eau (65-25-10). Les fractions les moins polaires contiennent alors moins de 2 % du poids de plante mise en œuvre.

L'extrait conforme à la présente invention, c'est-à-dire le produit B a ensuite été soumis à des analyses physicochimiques qui ont permis de relever les constatations suivantes :

— produit pâteux, de couleur jaune brunâtre,
— solubilités : éther, chloroforme, éthanol,
— C.C.M. : silice, chloroforme, méthanol 9-1.

Principales taches : Rf = 0,93-0,87-0,80-0,75-0,53-0,3-0,25-0,20-0,05.
— identification du composé de Rf = 0,53 :

PM = 254
Formule brute : $C_{16}H_{14}O_3$
$\alpha_D^{22}$ = — 43° (C = 1, $CH_3OH$)
I.R = 3 400, 2 900, 1 610, 1 505 $cm^{-1}$
U.V = 219, 266, 303 nm
Masse = $M^+$ = 254 — fragments à 147 ($C_9H_7O_2$) et 107 ($C_7H_7O$)

RMN dans $CD_3OD$ :

— 7 H aromatiques entre 6,3 et 7,1 ppm
— 1 H (doublet J = 12 Hz) à 6,3 ppm
— 1 H (doublet de doublet, $J_1$ = 12 Hz, $J_2$ = 5 Hz) à 5,7 ppm
— 2 H (singulet élargi) à 4,95 ppm
— 2 H (multiplet) à 4,1 ppm
— 1 H (multiplet) à 3,80 ppm ;

RMN dérivé diacétylé dans $CDCl_3$ :

— 7 H aromatiques entre 6,6 et 7,3 ppm
— 1 H (doublet, J = 12 Hz) à 6,4 ppm
— 1 H (doublet de doublet, $J_1$ = 12 Hz, $J_2$ = 4 Hz) à 5,9 ppm
— 2 H (multiplet) à 4,2 ppm
— 1 H (multiplet) à 3,9 ppm
— 6 H (singulet) à 2,2 ppm.

RMN $^{13}C$ (dérivé acétylé) ($CDCL_3$ · δTMS = 0) :

20,86 (2 q) 49,4 (d) 74,58 (t) 113,24 (d) 115,77 (d) 121,48 (2 d) 124,19 (s) 127,90 (d) 129,25 (2 d) 132,31 (d) 133,13 (d) 138,14 (s) 150,0 (2 s) 159,56 (s) 169,03 (s) 169,27 (s).
$[\alpha_D]^{20}$ (C = 1 %, $CH_3OH$) = — 43°.

L'ensemble des caractéristiques physicochimiques relevées précédemment ont permis de conclure à la présence de ruscobenzoxépine de formule

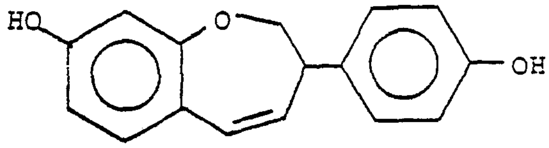

Pharmacologie

Afin de quantifier l'activité veinotonique des produits A et B selon l'exemple précédent, nous avons utilisé le test d'Anneaux de veines Saphène isolés, *in vitro,* suspendus dans du liquide de Krebs-Ringer. La standardisation est faite en calculant la contraction, induite par les produits A et B, en pourcentage de la contraction induite par une dose constante de Noradrenaline ($3 \cdot 10^{-7}$M) correspondante à 50 % de la contraction maximale de la veine ($ED_{50NA}$ = 9,13 g ± 2,05 g).

| Conc.* / Produit | 1 | 2 | 3 | 4 | 5 |
|---|---|---|---|---|---|
| A | 0 | 2 | 4 | 16 | 17 |
| B | 0 | 17,5 | 31 | 32 | 33 |

Conc.* : Concentration de produit (en mg) ajouté dans la cuve (50 ml) de façon cumulative.

Le produit B permet donc une amélioration nette de la veinotonicité par rapport au produit A de plus de 40 % en moyenne.

Compte tenu des excellentes propriétés veinotoniques de l'extrait de Ruscus conforme à la présente invention, ce dernier peut avantageusement être utilisé en tant que principe actif dans diverses compositions tant à usage pharmacologique qu'à usage cosmétologique.

**Revendications**

1. Procédé de préparation d'un extrait de Ruscus (Liliacées), en particulier de Ruscus aculeatus, caractérisé par le fait qu'il comporte au moins une première étape d'extraction par un alcool ou un mélange hydroalcoolique, suivie d'une évaporation et d'une seconde étape d'extraction par un ester ou une cétone, en vue d'éliminer les saponines stéroliques présentes dans l'extrait de ladite première étape.

2. Procédé selon la revendication 1, caractérisé en ce que les alcools utilisés pour la première étape sont choisis parmi les alcanols en $C_1$ à $C_7$.

3. Procédé selon l'une des revendications 1 et 2, caractérisé en ce que les esters utilisés pour la deuxième étape sont choisis parmi les acétates d'éthyle et d'isopropyle, et les cétones parmi la diméthylcétone, la méthyléthylcétone ou la méthylisobutylcétone.

4. Procédé selon l'une des revendications 1 à 3, caractérisé par une purification chromatographique complémentaire sur silice ou alumine visant à l'isolement des fractions les moins polaires.

5. Procédé selon l'une des revendications 1 à 4, caractérisé par une étape de purification complémentaire visant à enrichir l'extrait en composés phénoliques, par résines échangeuses d'ions, poudre de polyamide ou polyvinylpyrrolidone.

6. Procédé selon l'une des revendications 1 à 5, caractérisé par le fait que ladite évaporation du solvant alcoolique ou hydroalcoolique contenant le principe actif est réalisée sur une matière inerte et/ou adsorbante telle que la silice, l'alumine, un carbonate alcalin ou alcalino-terreux, des sucres, du kaolin, de la cellulose ou de cyclodextrine.

**Claims**

1. Process for preparing a Ruscus extract (Liliaceae), more particularly of Ruscus aculeatus, characterised in that the said process comprises at least one first stage of extraction by an alcohol or a hydroalcoholic mixture, followed by an evaporation and a second extraction stage by an ester or a ketone with a view to eliminating the sterolic saponins, which are present in the extract of the said first stage.

2. Process according to claim 1, characterised in that the alcohols used for the first stage are selected from amongst the $C_1$ to $C_7$ alkanols.

3. Process according to either claim 1 or 2, characterised in that the esters used for the second stage are selected from amongst the ethyl and isopropyl acetates, and the ketones are selected from dimethyl ketone, methylethyl ketone or methylisobutyl ketone.

4. Process according to any one of claims 1 to 3, characterised by an additional chromatographic purification on silica or alumina with a view to isolating the fractions having the least polarity.

5. Process according to any one of claims 1 to 4, characterised by an additional purification stage with a view to enriching the extract with phenolic compounds, by means of ion-exchange resins, polyamid powder or polyvinyl pyrrolidone.

6. Process according to any one of claims 1 to 5, characterised in that the said evaporation of the alcoholic or hydroalcoholic solvent containing the active principle is effected on an innert and/or absorbant substance such as silica, alumina, an alkaline or alkaline-earth carbonate, sugars, kaolin, cellulose or cyclodextrin.

**Patentansprüche**

1. Verfahren zur Herstellung eines Ruscusextraktes (Liliaceae), insbesondere aus Ruscus aculeatus, dadurch gekennzeichnet, daß ein erster Extraktionsscchritt mit Hilfe eines Alkohols oder eines wäßrigen Alkohols mit darauffolgendem Verdampfen und ein zweiter Extraktionsschritt mit Hilfe eines Esters oder eines Ketons erfolgt, um die im Extrakt des genannten ersten Schrittes enthaltenen steroidischen Saponine zu eliminieren.

2. Verfahren nach Anspruch 1, dadurch gekennzeichnet, daß die für den ersten Schritt verwendeten Alkohole aus Alkanolen mit 1 bis 7 C-Atomen ausgewählt werden.

3. Verfahren nach einem der Ansprüche 1 und 2, dadurch gekennzeichnet, daß die für den zweiten Schritt verwendeten Ester aus Ethyl- und Isopropylacetaten, und die Ketone aus Dimethylketon, Methylethylketon oder Methylisobutylketon ausgewählt werden.

4. Verfahren nach einem der Ansprüche 1 bis 3, gekennzeichnet durch eine zusätzliche chromatographische Reinigung auf Silika oder Tonerde, um die am wenigsten polaren Fraktionen zu isolieren.

5. Verfahren nach einem der Ansprüche 1 bis 4, gekennzeichnet durch einen zusätzlichen Reinigungsschritt, um das Extrakt mit Phenolverbindungen durch Ionenaustauschharze, Polyamidpulver oder Polyvinylpyrrolidon anzureichern.

6. Verfahren nach einem der Ansprüche 1 bis 5, dadurch gekennzeichnet, daß das genannte Verdampfen des alkoholischen oder wäßrigen alkoholischen, den aktiven Bestandteil enthaltenden Lösungsmittels auf einem inerten und/oder adsorbierenden Stoff, wie z. B. Silika, Tonerde, Alkali- oder Erdalkalicarbonat, Zucker, Kaolin, Zellulose oder Cyclodextrin, erfolgt.